# EUROPÄISCHE PATENTSCHRIFT

(11) **EP 0 556 210 B1**
(45) Veröffentlichungstag und Bekanntmachung des Hinweises auf die Patenterteilung: **25.01.1995**
(21) Anmeldenummer: 91918490.3
(22) Anmeldetag: 02.11.1991
(51) Int. Cl.: A61B 17/34, A61M 5/46

(54) **EPIDURALKANÜLE**
EPIDURAL CANNULA
CANULE EPIDURALE

(30) Priorität: 05.11.1990 ES 9002795
(43) Veröffentlichungstag der Anmeldung: 25.08.1993
(73) Patentinhaber: WASKÖNIG, Wilhelm, F-93140 Bondy (FR); RODIERA OLIVE, José Javier, 08022 Barcelona (ES)
(72) Erfinder: WASKÖNIG, Wilhelm, F-93140 Bondy (FR); RODIERA OLIVE, José Javier, 08022 Barcelona (ES)
(74) Vertreter: Stoffregen, Hans-Herbert, Dr. Dipl.-Phys.
(86) Internationale Anmeldenummer: EP9102069
(87) Internationale Veröffentlichungsnummer: WO9207520

(56) Entgegenhaltungen:
- US-A- 2 922 420
- US-A- 3 081 770

## Beschreibung

Die Erfindung bezieht sich auf eine Epiduralkanüle zur Anästhesie nach den Oberbegriffen der nebengeordneten Ansprüche.

Bei der Epiduralanästhesie muß die Spitze und die Öffnung zwischen dem Ligamentum Flavum (gelbes Band) und der Dura Mater Membran liegen. Mit üblichen, einen gleichbleibenden Querschnitt aufweisenden Nadeln (siehe z.B. US-A-2,922,420) verringert sich abrupt die notwendige Kraft beim Einstechen in dem Moment, wo die Kanülenspitze das Ligamentum Flavum durchstoßen hat, so daß die Gefahr einer ungewollten Perforation der Dura Mater Membran erwächst. Um dies zu vermeiden, muß der die Epiduralkanüle einsetzende Mediziner Geschick haben und Erfahrungen aufweisen.

Aus der US-A-3,081,770 ist eine für Operationen bestimmte Kanüle bekannt, deren Kanülenkörper im Abstand zur Kanülenspitze eine Querschnittsänderung aufweist. Der zwischen Querschnittsänderung und eine zentrale Öffnung aufweisender Kanülenspitze verlaufende Bereich ist im Querschnitt rechteckförmig oder oval.

Der vorliegenden Erfindung liegt die Aufgabe zugrunde, eine Epiduralkanüle der zuvor beschriebenen Art so weiterzubilden, daß weitgehend sichergestellt ist, daß eine ungewollte Perforation der Dura Mater Membran unterbleibt, daß also sichergestellt ist, daß sich die Kanülenspitze mit im Abstand zu dieser verlaufenden Öffnung innerhalb des Epiduralraums befindet, uni anästhesieren bzw. punktieren zu können.

Die Aufgabe wird erfindungsgemäß durch die den nebengeordneten Ansprüchen zu entnehmenden Maßnahmen gelöst. Diese zeichnen sich einerseits dadurch aus, daß der Kanülenkörper im Abstand zur Kanülenspitze eine Querschnittserweiterung in Form einer wulstartigen Erweiterung aufweist, die zumindest einen der Kanülenspitze zugewandten Bereich aufweist, der zur Epiduralkanülenlängsachse betrachte in Richtung der Kanülenspitze einen Winkel α mit α > 30° aufweist, und daß der Abstand zwischen proximalem Punkt des Kanülenschliffs und dem der Kanülenspitze abgewandten Ende der wulstartigen Erweiterung in etwa der Dicke des Gelben Bandes ist. Andererseits ist vorgesehen, daß der Kanülenkörper im Abstand zur Kanülenspitze eine Querschnittserweiterung derart aufweist, daß bei auf die Epiduralkanüle in Richtung der Kanülenspitze einwirkender Kraft deren Vorwärtsbewegung dann abbremsbar ist, wenn die Erweiterung in das Gelbe Band eindringt, wobei die Erweiterung zumindest abschnittsweise durch einen zur Längsachse der Epiduralkanüle betrachtet in Richtung der Kanülenspitze unter einem Winkel α mit α > 30° verlaufenden Kanülenkörperwandabschnitt gebildet ist und wobei der Abstand zwischen dem freien vorderen Ende der Kanülenspitze und dem Beginn der Erweiterung gleich oder kleiner als der Abstand zwischen Gelbem Band und Dura Mater Membran auf Lumbar-Höhe ist. Ausgestaltungen ergeben die Unteransprüche.

Erfindungsgemäß erfolgt durch vorzugsweise eine abrupte Querschnittsänderung, die nicht notwendigerweise umlaufend und/oder symmetrisch zur Längsachse des Kanülenkörpers verlaufen muß, ein Aufbau eines der Bewegung entgegengerichteten Widerstandes dann, wenn die Erweiterung das gelbe Band berührt bzw. dieses durchdringen soll. Die zum weiteren Vortreiben der Nadel erforderliche Kraft nimmt ferner progressiv mit der Penetration zu, so daß - im Vergleich zu den bekannten Epiduralnadeln - keine Kraftentlastung dann erfolgt, wenn das Ligamentum Flavum durchdrungen wird. Folglich ist eine Perforation der Dura Mater Membran nahezu ausgeschlossen.

Auch wenn bereits Kanülen bekannt sind, die entlang ihrer Längsachse unterschiedliche Durchmesser aufweisen (siehe z. B. EP-A-0 359 987, US-A-3,081,770, US-A-3,540,447), so soll hierdurch gerade nicht das der Erfindung zugrundeliegende Problem gelöst werden, eine Perforation der Dura Mater Membran zu vermeiden, sondern durch die gewählte Geometrie sollen Komplikationen beim Durchstechen der Dura Mater Membran verringert werden. Folglich will man mit den angesprochenen Kanülen gerade die Dura Mater Membran durchstechen und gleichzeitig sicherstellen, daß das Liquorleck-Syndrom weitgehend vermieden wird.

Um eine eindeutige Lagepositionierung der seitlichen Öffnung der Epiduralnadel zwischen Gelbem Band und Dura Mater Membran sicherzustellen, entspricht der Abstand zwischen dem freien vorderen Ende Kanülenspitze und dem Beginn der Querschnittsänderung des Epidiuralkanülenkörpers dem Abstand zwischen dem Gelben Band und der Dura Mater Membran auf Lumbar-Höhe.

Unter Abstand hierbei wird nicht notwendigerweise nur der natürliche Abstand zwischen Gelbem Band und Dura Mater Membran verstanden, sondern auch derjenige, der dann - allerdings ohne Perforation der Dura Mater Membran - vorliegen kann, wenn die Kanülenspitze gegen die Dura Mater Membran stößt, diese also berührt und dehnt. Bei der Ausbildung der Erweiterung in Form des Wulstes ist dessen Erstreckung in Richtung der Längsachse der Epiduralkanüle gleich oder kleiner als die Dicke des Gelben Bandes, so daß der Wulst vollständig im Gelben Band liegt, wenn sich der Kanülenschliff im Epiduralraum befindet. Hierdurch ist eine eindeutige Positionierung der Kanülenöffnung im Epiduralraum sichergestellt, so daß auch unkontrollierte Stoß- bzw. Zugeinwirkungen auf die Epiduralkanüle zu einer Positionsveränderung nicht führen können.

Bevorzugterweise beträgt die Differenz zwischen dem Durchmesser der Epiduralkanüle vor der Erweiterung und der Erweiterung selbst maximal zwei Gauge, vorzugsweise ein Gauge. Durch diese Querschnittsänderungen wird das erfindungsgemäße Ziel erreicht, daß sich der Bremseffekt durch die Querschnittsänderung derart bemerkbar macht, daß beim weiteren Vordringen der Epiduralkanüle eine Perforationsgefahr der Dura Mater Membran und die damit verbundenen Komplikationen vermieden werden.

Vorteilhaft kann es des weiteren sein, wenn die Öffnung der Epiduralkanüle mit ihrem der Erweiterung zugewandten Bereich zu diesem einen Abstand aufweist, der in etwa gleich der Dicke des Gelben Bandes ist.

Zu der Kanülenspitze selbst ist anzumerken, daß diese bekannte Schliffe wie den Hustead- oder Tuohy-Schliff aufweisen kann.

Sofern die Erweiterung nicht umlaufend ausgebildet ist, sollte deren periphere Erstreckung vorzugsweise über 2/3 des Kanülenkörperumfangs erfolgen.
Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: einen Längsschnitt durch eine erste Ausführungsform einer Epiduralkanüle,
- Fig. 2: einen Längsschnitt durch eine zweite Ausführungsform einer Epiduralkanüle,
- Fig. 3: die Epiduralkanüle gemäß Fig. 1 mit zwischen Gelbem Band und Dura Mater Membran angeordneter Kanülenöffnung und
- Fig. 4: die der Fig. 2 zu entnehmende Epiduralkanüle mit zwischen Gelbem Band und Dura Mater Membran angeordneter Kanülenöffnung.

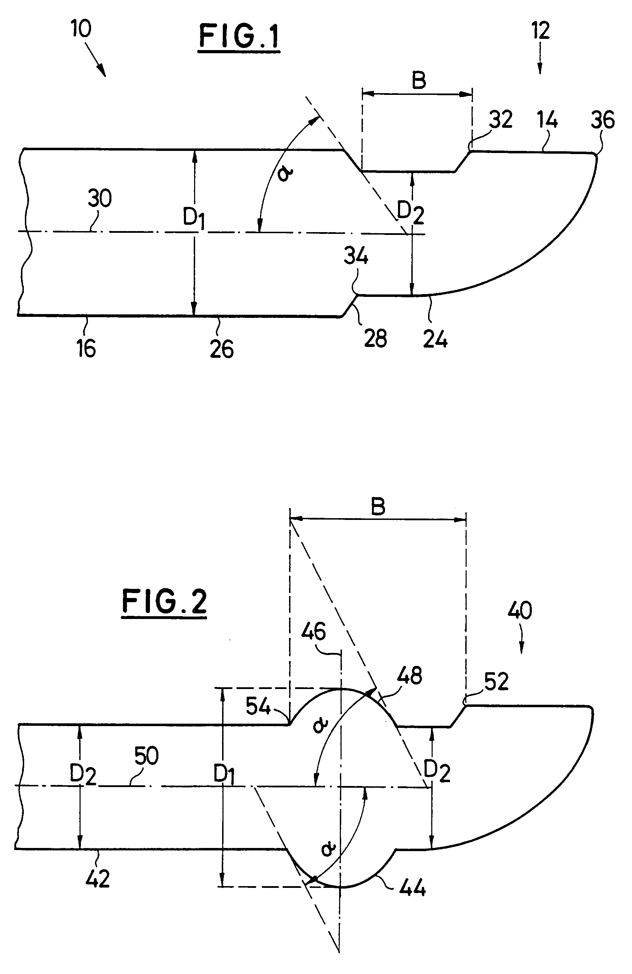

In Fig. 1 ist rein prinzipiell eine erste Ausführungsform einer Epiduralkanüle (10) dargestellt, die eine Kanülenspitze (12) mit seitlicher Öffnung bzw. Schliff (14) und einen von einer nichtdargestellten Halterung aufgenommenen Kanülenkörper (16) aufweist. Insoweit handelt es sich grundsätzlich um eine bekannte Kanüle.

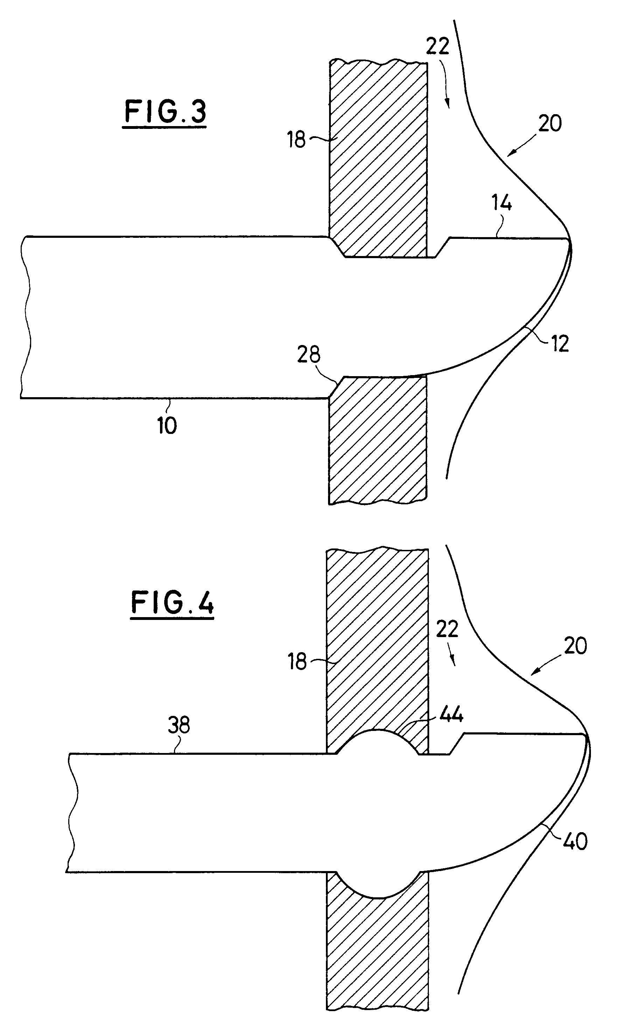

Erfindungsgemäß ist nun vorgesehen, daß zwischen dem Kanülenkörper (16) und der Kanülenspitze (12) eine Querschnittsänderung erfolgt, die dazu führt, daß die Epiduralkanüle (10) beim Durchstoßen eines Gelben Bandes (18) (Fig. 3) abgebremst wird. Hierdurch wird beim weiteren Durchdringen des Gelben Bandes (18) eine im wesentlichen progressive Kraftänderung hervorgerufen. Somit wird sichergestellt, daß ein unkontrolliertes Durchstoßen bzw. Perforieren einer Dura Mater Membran (20) unterbleibt, wodurch andemfalls das bekannte Liquorleck-Syndrom auftreten könnte.

Mit anderen Worten soll durch die Querschnittsänderung sichergestellt sein, daß die Kanülenspitze (12) mit ihrer Öffnung (14) eindeutig im Epiduralraum (22) positionierbar ist, um dort anästhesieren zu können.

Die Querschnittsänderung wird in Fig. 1 durch die voneinander abweichenden Durchmesser D₂ und D₁ rein prinzipiell angegeben. Der Übergang zwischen den voneinander abweichenden Durchmessein, also zwischen dem Bereich (24) der Kanülenspitze (12) mit dem Durchmesser D₂ und dem Bereich (26) des Kanülenkörpers (16) mit dem Durchmesser D₁ kann dabei stetig, jedoch abrupt erfolgen, wie die Fig. 1 verdeutlichen soll. Dieser Übergang ist mit dem Bezugszeichen (28) versehen und weist eine Kegelstumpfgeometrie auf.

Der Kanülenkörperwandabschnitt, also der Übergang (28) beschreibt dabei zu der Epiduralkanülenkörperlängsachse (30) einen Winkel α, der vorzugsweise im Bereich zwischen 30° und 60°, vorzugsweise bei 45° liegt. Hierdurch erfolgt eine hinreichende Bremswirkung dann, wenn der Übergang (28), also die Querschnittserweiterung gegen das Gelbe Band (18) stößt. Dies sollte dann erfolgen, wenn der Schliff der Kanülenspitze (12) das Gelbe Band (18) durchstoßen hat. Daher sollte der Abstand B des proximalen Punktes (32) des Nadelschliffs bis zu dem Punkt (34), in dem die Querschnittsänderung erfolgt, in etwa gleich der Breite des Gelben Bandes (18) sein.

In Fig. 2 ist eine Epiduralnadel (38) dargestellt, die gleichfalls eine Kanülenspitze (40) und einen Kanülenkörper (42) aufweist, wobei jedoch der im Bereich der Spitze vorhandene Durchmesser D₂ auch im Bereich des übrigen Kanülenkörpers (42) gegeben ist, sofern von der umlaufenden und als Wulst zu bezeichnenden Erweiterung (44) absieht, die eine Querschnittsänderung hervorruft, die der in Fig. 1 (Bezugszeichen 28) von der Funktion her entspricht.

Die wulstartige Erweiterung (44) oder Ausbauchung, die im Ausführungsbeispiel einen zur Querschnittsebene (46) symmetrischen Verlauf zeigt, weist im der Kanülenspitze (44) zugewandten Abschnitt (48) zumindest bereichsweise eine Neigung auf, die einen Winkel α zur Epiduralkanülenlängsachse (50) beschreiben sollte, der gleich dem der Epiduralkanüle (10) entspricht.

Die in Längsrichtung der Epiduralkanüle (38) gegebene Erstreckung der Querschnittserweiterung (44) sollte so gewählt werden, daß zwischen proximalem Punkt (52) des Schliffes der Kanülenspitze (40) und dem der Spitze (40) abgewandten Ende (54) ein Abstand B gegeben ist, der gleich der Dicke des Gelben Bandes (18) ist.Hierdurch ist sichergestellt, daß dann, wenn sich der Schliff der Kanülenspitze (40) und damit die Öffnung im Epiduralraum (22) befindet, die Erweiterung (44) vollständig in dem Gelben Band (18) lagefixiert ist, so daß unkontrollierte Stoß- oder Zugeinwirkungen auf die Epiduralkanüle (38) zu keiner Lageveränderung führen.

Bei den Schliffen der Epiduralkanülen (10) bzw. (38) kann es sich um übliche handeln, also um einen Hustead- oder Tuohy-Schliff.

Zu den Abmessungen der erfindungsgemäßen Epiduralkanüle (10) bzw. (38) ist folgendes anzumerken.

Der Abstand zwischen dem proximalen Punkt (32) bzw. (52) des Schliffes und dem Beginn der Querschnittsänderung (28) bzw. (44) sollte in der Größenordnung von 2 mm liegen.

Hinsichtlich der Querschnittsverhältnisse - einerseits im Bereich der Kanülenspitze (12) bzw. (40), andererseits der Erweiterung (26) bzw. (44) - sollte eine Differenz von maximal 2 Gauge, vorzugsweise 1 Gauge bestehen, wobei der Durchmesser D₂ vorzugsweise 19 bzw. 18 Gauge (1,00 mm bzw. 1,20 mm) und der Durchmesser D₁ 18 bzw. 17 Gauge (1,20 mm bzw. 1,40 mm) beträgt.

## Patentansprüche

1. Epiduralkanüle (38) zur Anästhesie umfassend eine einen Kanülenschliff aufweisende Kanülenspitze (40) mit in deren Bereich vorhandener Öffnung sowie einen zentralen, vorzugsweise eine Zylinderform aufweisenden Kanülenkörper (42), dessen der Spitze abgewandtes Ende mit einer Halterung verbindbar ist, wobei bei der Nutzung der Epiduralkanüle deren Öffnung zwischen Gelbem Band (18) und Dura Mater Membran (20) positionierbar ist,
**dadurch gekennzeichnet**,
daß der Kanülenkörper (42) im Abstand zur Kanülenspitze (40) eine Querschnittserweiterung (44) in Form einer wulstartigen Erweiterung aufweist, die zumindest einen der Kanülenspitze (40) zugewandten Bereich (48) aufweist, der zur Epiduralkanülenlängsachse (50) betrachtet in Richtung der Kanülenspitze einen Winkel α mit α > 30° aufweist, und daß der Abstand (B) zwischen proximalem Punkt (52) des Kanülenschliffs und dem der Kanülenspitze (40) abgewandten Ende (54) der wulstartigen Erweiterung (44) in etwa der Dicke des Gelben Bandes (18) ist.

2. Epiduralkanüle nach Anspruch 1,
**dadurch gekennzeichnet**,
daß die wulstartige Erweiterung in bezug auf eine Querschnittsebene (46) symmetrisch ausgebildet ist.

3. Epiduralkanüle nach Anspruch 1 oder 2,
**dadurch gekennzeichnet**,
daß die Epiduralkanüle (38) im Bereich ihrer Spitze (40) einen Durchmesser von vorzugsweise 1,00 mm (19 Gauge) bzw. 1,20 mm (18 Gauge) und die Erweiterung (44) einen maximalen Durchmesser von vorzugsweise 1,20 mm (18 Gauge) bzw. 1,40 mm (17 Gauge) aufweist.

4. Epiduralkanüle nach zumindest einem der vorhergehenden Ansprüche,
**dadurch gekennzeichnet**,
daß der Abstand zwischen dem freien vorderen Ende (36) der Kanülenspitze (40) und dem Beginn der Erweiterung (44) gleich oder kleiner als der Abstand zwischen Gelbem Band (18) und Dura Mater Membran (20) auf Lumbar-Höhe ist.

5. Epiduralkanüle (10, 38) zur Anästhesie umfassend eine Kanülenspitze (12, 40) mit seitlicher Öffnung (14) und Kanülenschliff sowie einen zentralen, vorzugsweise eine Zylinderform aufweisenden Kanülenkörper (16, 42), dessen der Kanülenspitze abgewandtes Ende mit einer Halterung verbindbar ist, wobei bei der Nutzung der Epiduralkanüle deren Spitze mit Öffnung zwischen Gelbem Band (18) und Dura Mater Membran (20) positionierbar ist,
**dadurch gekennzeichnet**,
daß der Kanülenkörper (16, 42) im Abstand zur Kanülenspitze (12, 40) eine Querschnittserweiterung (28, 44) derart aufweist, daß bei auf die Epiduralkanüle (10, 38) in Richtung der Kanülenspitze einwirkender Kraft deren Vorwärtsbewegung dann abbremsbar ist, wenn die Erweiterung in das Gelbe Band (18) eindringt, wobei die Erweiterung (28, 44) zumindest abschnittsweise durch einen zur Längsachse (30,50) der Epiduralkanüle (10, 38) betrachtet in Richtung der Kanülenspitze unter einem Winkel α mit α > 30° verlaufenden Kanülenkörperwandabschnitt (28) gebildet ist und wobei der Abstand zwischen dem freien vorderen Ende (36) der Kanülenspitze (12, 40) und dem Beginn (34) der Erweiterung (28, 44) gleich oder kleiner als der Abstand zwischen Gelbem Band (18) und Dura Mater Membran (20) auf Lumbar-Höhe ist.

6. Epiduralkanüle nach zumindest einem der vorhergehenden Ansprüche,
**dadurch gekennzeichnet**,
daß die Differenz zwischen dem Durchmesser (D₂) der Epiduralkanüle (10, 38) vor deren Erweiterung (28, 44) und der Erweiterung selbst maximal 0,4 mm (2 Gauge), vorzugsweise 0,2 mm (1 Gauge) beträgt.

7. Epiduralkanüle nach zumindest einem der vorhergehenden Ansprüchen,
**dadurch gekennzeichnet**,
daß die Erweiterung (28, 44) zumindest bereichsweise umlaufend ausgebildet ist, wobei deren periphere Erstreckung über vorzugsweise zwei Drittel des Umfangs des Kanülenkörpers (16,42) erfolgt.

8. Epiduralkanüle nach zumindest einem der vorhergehenden Ansprüche,
**dadurch gekennzeichnet**,
daß die Kanülenspitze (12, 40) vorzugsweise einen Hustead- oder Tuohy-Schliff aufweist.

## Claims

1. An epidural cannula (38) for anesthesia, comprising a cannula point (40) having a microsection with an opening in its vicinity and a central, preferably cylinder-shaped cannula body (42) of which the end away from said point is connectable to a holding means, where said opening is positionable during the use of said epidural cannula between the ligamenta flava (18) and the dura mater (20),
**wherein**
said cannula body (42) has a cross-sectional enlargement (44) at a distance from said cannula point (40) in the form of a bulge-like enlargement having at least one area (48) towards said cannula point (40) that describes an angle α with α > 30° in relation to said epidural cannula longitudinal axis (50), seen in the direction of said cannula point, and wherein the distance (B) between the proximal point (52) of said cannula microsection and the end (54) of said bulge-like enlargement (44) away from said cannula point (40) is approximately as thick as said ligamenta flava (18).

2. An epidural cannula according to Claim 1,
**wherein**
said bulge-like enlargement is designed symmetrical in relation to a cross-sectional plane (46).

3. An epidural cannula according to Claim 1 or 2,
**wherein**
said epidural cannula (38) has a diameter in the area of its point (40) of preferably 1,00 mm (19 gauges) or 1,2 mm (18 gauges) and said enlargement (44) a maximum diameter of preferably 1,20 mm (18 gauges) or 1,40 mm (17 gauges).

4. An epidural cannula according to at least one of the preceding claims,
**wherein**
the distance between the free front end (36) of said cannula point (40) and the start of said enlargement (44) is identical to or smaller than the distance between said ligamenta flava (18) and said dura mater (20) at the lumbar level.

5. An epidural cannula (10, 38) for anesthesia, comprising a cannula point (12, 40) with a lateral opening (14) and microsection, and a central, preferably cylinder-shaped cannula body (16, 42) of which the end away from said cannula point is connectable to a holding means, where said opening of said point of said epidural cannula is positionable during the use of said epidural cannula between the ligamenta flava (18) and the dura mater (20),
**wherein**
said cannula body (16, 42) has a cross-sectional enlargement (28, 44) at a distance from said cannula point (12, 40) such that when a force is exerted on said epidural cannula (10, 38) in the direction of its point the forward motion of said cannula can be stopped when said enlargement enters said ligamenta flava (18), where said enlargement (28, 44) is formed at least in sections by a cannula body wall section (28) at an angle α in relation to said longitudinal axis (30, 50) of said epidural cannula (10, 38), seen in the direction of said cannula point, with α > 30°, and where the distance between the free front end (36) of said cannula point (12, 40) and the start (34) of said enlargement (28, 44) is identical to or smaller than the distance between said ligamenta flava (18) and said dura mater (20) at the lumbar level.

6. An epidural cannula according to at least one of the preceding claims,
**wherein**
the difference between the diameter (D₂ of said epidural cannula (10, 38) in front of said enlargement (28, 44) thereof and that of said enlargement itself is a maximum of 0,4 mm (2 gauges), preferably 0,2 mm (1 gauge).

7. An epidural cannula according to at least one of the preceding claims,
**wherein**
said enlargement (28, 44) is of all-round design at least in some areas, where it peripherally extends preferably over two thirds of the circumference of said cannula body (16, 42).

8. An epidural cannula according to at least one of the preceding claims,
**wherein**
said cannula point (12, 40) preferably has a Hustead or Tuohy microsection.

## Revendications

1. Canule épidurale (38) pour l'anesthésie comprenant une pointe de canule (40) présentant une section polie de canule avec un orifice présent dans cette zone ainsi qu'un corps de canule (42) centrale présentant une forme cylindrique de préférence dont l'extrémité opposée à la pointe peut être reliée à un support, et lors de l'utilisation de la canule épidurale on peut positionner son orifice entre la bande jaune (18) et la membrane dure mère (20), caractérisé en ce que le corps de canule (42) présente à une certaine distance de la pointe de canule (40) un élargissement de section (44) sous l'aspect d'un élargissement en forme de bourrelet, qui présente au moins une zone (48) dirigée vers la pointe de canule (40), qui fait un angle α, avec α > 30°, par rapport à l'axe longitudinal de canule épidurale (50) dans le sens de la pointe de canule et que la distance (B) entre le point (52) le plus proche de la section polie de canule et l'extrémité (54) opposée à la pointe de canule (40) de l'élargissement (44) en forme de bourrelet correspond approximativement à l'épaisseur de la bande jaune (18.

2. Canule épidurale selon la revendication 1, caractérisée en ce que l'élargissement en forme de bourrelet est symétrique par rapport à un plan de section (46).

3. Canule épidurale selon la revendication 1 ou 2, caractérisée en ce que la canule épidurale (38) présente dans la zone de sa pointe (40) un diamètre ayant de préférence 1,00 mm (calibre 19) ou 1,20 mm (calibre 18) et l'élargissement présente un diamètre maximal de 1,20 mm (calibre 18) de préférence ou de 1,40 mm (calibre 17).

4. Canule épidurale selon au moins une des revendications précédentes caractérisée en ce que la distance entre l'extrémité antérieure libre (36) de la pointe de canule (40) et le début de l'élargissement (44) est égale ou inférieure à la distance entre la bande jaune (18) et la membrane dure mère (20) à la hauteur des lombaires.

5. Canule épidurale (10, 38) pour l'anesthésie comprenant une pointe de canule (12, 40) avec un orifice latéral (14) et une section polie de canule, ainsi qu'un corps de canule (16, 42) central présentant une forme cylindrique, dont l'extrémité opposée à la pointe de canule peut être reliée à un support, et lors de l'utilisation de la canule épidurale on peut positionner sa pointe avec l'orifice compris entre la bande jaune (18) et la membrane dure mère (20), caractérisée en ce que le corps de canule (16, 42) présente à une certaine distance de la pointe de canule (12, 40) un élargissement de section (28, 44), de sorte qu'une force s'exerçant dans le sens de la pointe de canule sur la canule épidurale (10, 38) se trouve freinée, quand l'élargissement pénètre dans la bande jaune (18), l'élargissement (28, 44) étant formé au moins en partie par une partie de paroi (28) de corps de canule qui fait un angle α avec α > 30° par rapport à l'axe longitudinal de canule (30, 50) de la canule épidurale (10, 38) dans le sens de la pointe de canule et que la distance entre l'extrémité libre (36) antérieure de la pointe de canule (12, 40) et le début (34) de l'élargissement (28, 44) est égale ou inférieure à la distance entre la bande jaune (18) et la membrane dure mère (20) à la hauteur des lombaires.

6. Canule épidurale selon au moins une des revendications précédentes, caractérisée en ce que la différence entre le diamètre (D₂) de la canule épidurale (10, 38) avant son élargissement (28, 44) et l'élargissement lui-même représente au maximum 0,4 mm (calibre 2), de préférence 0,2 mm (calibre 1).

7. Canule épidurale selon au moins une des revendications précédentes, caractérisée en ce que l'élargissement (28, 44) est constitué suivant un cercle au moins partiellement, son extension périphérique s'étendant de préférence sur les deux tiers de la périphérie du corps de canule (16, 42).

8. Canule épidurale selon au moins l'une des revendications précédentes, caractérisée en ce que la pointe de canule (12, 40) présente de préférence une section polie de Hustead ou de Tuohy.
